(19) **Europäisches Patentamt / European Patent Office / Office européen des brevets**

(11) **EP 2 635 608 B1**

(12) **FASCICULE DE BREVET EUROPEEN**

(45) Date de publication et mention de la délivrance du brevet:
**11.03.2026 Bulletin 2026/11**

(21) Numéro de dépôt: **11802459.5**

(22) Date de dépôt: **02.11.2011**

(51) Classification Internationale des Brevets (IPC):
**C08B 30/04** (2006.01)    **A61P 7/08** (2006.01)
**C08B 30/18** (2006.01)    **C12P 19/04** (2006.01)
**C12P 19/14** (2006.01)    **A61K 31/718** (2006.01)
**B01D 61/14** (2006.01)

(52) Classification Coopérative des Brevets (CPC):
**C08B 30/04; A61K 31/718; A61P 7/08; B01D 61/14; C08B 30/18; C12P 19/04; C12P 19/14**

(86) Numéro de dépôt international:
**PCT/FR2011/052555**

(87) Numéro de publication internationale:
**WO 2012/059685 (10.05.2012 Gazette 2012/19)**

(54) **PROCÉDÉ DE DÉCONTAMINATION D'HYDROLYSATS D'AMIDON POUR LA PRÉPARATION DE POLYMÈRES DE GLUCOSE DESTINÉS À LA DIALYSE PÉRITONÉALE**

VERFAHREN ZUR REINIGUNG VON STÄRKE-HYDROLYSATEN ZUR HERSTELLUNG VON GLUCOSE-POLYMEREN FÜR DIE PERITONEALE DIALYSE

PROCESS FOR THE DECONTAMINATION OF STARCH HYDROLYSATES FOR THE PREPARATION OF GLUCOSE POLYMERS FOR PERITONEAL DIALYSIS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **03.11.2010 FR 1059060**

(43) Date de publication de la demande:
**11.09.2013 Bulletin 2013/37**

(73) Titulaire: **Roquette Frères**
**62136 Lestrem (FR)**

(72) Inventeurs:
• **DUFLOT, Pierrick**
  **F-62136 La Couture (FR)**
• **PASSE, Damien**
  **F-59500 Douai (FR)**
• **VERRIN, Jean-Marc**
  **F-62660 Beuvry (FR)**

(74) Mandataire: **Plasseraud IP**
**104 Rue de Richelieu**
**CS92104**
**75080 Paris Cedex 02 (FR)**

(56) Documents cités:
**WO-A1-2007/099212    WO-A1-2010/125315**
**WO-A2-2009/117558**

EP 2 635 608 B1

**Description**

La présente invention se rapporte à un procédé de décontamination des hydrolysats d'amidon à partir desquels seront préparés des polymères de glucose destinés à la fabrication de solutions pour dialyse péritonéale.

On entend au sens de l'invention par « procédé de décontamination », un procédé qui permet de débarrasser les hydrolysats d'amidon de microorganismes contaminants (microorganismes vivants et/ou sporulés de type levures, moisissures et bactéries) et de substances susceptibles d'occasionner des péritonites (aseptiques ou non), complications majeures de la dialyse péritonéale, ces substances pouvant être :

- les lipopolysaccharides (LPS), complexes macromoléculaires toxiques présents de manière constitutive dans la membrane externe de toutes les bactéries à Gram négatif. Sur le plan structural, les LPS sont constitués d'un lipide A et d'une partie polysaccharidique débordant la membrane externe. Le lipide A est doué de propriétés toxiques et il correspond à l'endotoxine des bactéries à Gram négatif qui n'est libérée, de manière massive, qu'après lyse de la bactérie.
- des β-glucanes, polymères de D-glucose liés par des liaisons β-glucosidiques. Les β-glucanes sont un groupe diversifié de molécules, de masse moléculaire, solubilité, viscosité, et configuration tridimensionnelles variables. Les β-glucanes sont notamment constitutifs de la paroi cellulaire des cellules végétales, des levures et de certaines moisissures et bactéries,
- des peptidoglycanes (PGs), composants polysaccharidiques des parois des bactéries Gram (+). Encore appelés muréines, ou mucocomplexes, ou mucopeptides, les petidoglycanes sont formés d'une partie polysaccharide et d'une partie peptidique. Le polysaccharide est un polymère de glycosaminopeptide où la N-acétyl-glucosamine et l'acide N-acétyl-muramique sont liés par des liaisons osidiques β 1-4.

L'invention porte plus particulièrement sur la modification de la conduite des procédés classiques de production des hydrolysats d'amidon, au sens où sont ajoutées audits procédés classiques des étapes de filtration en série ou des étapes de traitement au charbon actif particulier, couplées ou non à des étapes de traitement enzymatique.

Ces étapes supplémentaires visent donc à garantir l'innocuité des hydrolysats d'amidon ainsi préparés, i.e. garantir une teneur en substances contaminantes bien inférieure au seuil de quantification des méthodes classiques de dosage desdites substances contaminantes.

La dialyse péritonéale est un type de dialyse qui a pour objectif d'éliminer les déchets tels que l'urée, la créatinine, l'excès de potassium ou l'excédent d'eau que les reins ne parviennent pas ou plus à épurer du plasma sanguin. Ce traitement médical est indiqué en cas d'insuffisance rénale chronique terminale.

C'est une épuration intracorporelle qui utilise le péritoine comme membrane de dialyse. Les déchets toxiques du sang traversent la membrane semi-perméable du péritoine, vers une solution appelée dialysat. Le dialysat est introduit dans la cavité péritonéale par un cathéter permanent. Il existe deux types de dialyse péritonéale :

- La DPCA (dialyse péritonéale continue ambulatoire), traitement qui se base sur le passage de 4 poches de dialysat par jour selon prescription médicale
- La DPA (dialyse péritonéale automatisée), traitement nocturne continu qui correspond à environ 15 litres de dialysat par 8 heures selon prescription médicale.

Les dialysats les plus couramment utilisés sont composés d'une solution tampon (du lactate ou du bicarbonate) à pH acide (5,2 - 5,5) ou physiologique (7,4) à laquelle sont ajoutés des électrolytes (sodium, calcium, magnésium, chlore) et un agent osmotique (du glucose ou un polymère de glucose, tel que l'« icodextrine » présent dans la solution pour dialyse péritonéale ambulatoire EXTRANEAL® commercialisée par la société BAXTER).

Les électrolytes et l'agent osmotique jouent chacun un rôle dans le mécanisme d'échange, selon leurs propriétés physico-chimiques respectives :

- les déchets du métabolisme (tels que l'urée ou la créatinine) ou autres électrolytes en surabondance que le rein n'élimine plus ou insuffisamment via l'appareil urinaire et les urines, vont s'extraire du plasma sanguin par diffusion des éléments vers le dialysat dont les taux de concentration de ces mêmes éléments sont moindres ;
- l'excédent d'eau, que le rein élimine normalement pour la régulation du volume plasmatique, va être attiré par osmolarité en fonction de la concentration du dialysat en glucose ou en polymère de glucose : plus la solution sera concentrée, plus l'eau présente dans le corps sera captée par le dialysat.

Le polymère de glucose, tel l'icodextrine mentionné ci-avant, est préféré au glucose comme agent osmotique, car bien que le glucose ait l'avantage d'être relativement sûr et peu coûteux, il a un certain nombre d'inconvénients.

En raison de sa petite taille, le glucose qui traverse rapidement le péritoine mène à la perte de gradient osmotique dans les 2 à 4 heures d'infusion.

Les caractéristiques d'ultrafiltration des solutions de dialyse péritonéale sont donc considérées meilleures en remplaçant le glucose par des substances de haut poids moléculaire, tels que des polymères de glucose, comme il sera démontré ci-après.

Les polymères de glucose standards sont produits par hydrolyse acide ou enzymatique d'amidon de céréales ou de tubercules.

L'hydrolyse acide de l'amidon, totalement aléatoire, ou son hydrolyse enzymatique un peu plus ordonnée, fournissent des mélanges de glucose (monomère) et des chaînes de glucose qui comportent des molécules très courtes (oligomères), de faible Degré de Polymérisation (ou D.P.), aussi bien que des molécules très longues (polymères), de D.P. élevé. Les polymères de glucose ont par ailleurs un poids moléculaire extrêmement varié.

Dans le domaine plus particulier de l'utilisation des polymères du glucose destinés à la dialyse péritonéale continue et ambulatoire, il est très vite apparu que ces hydrolysats d'amidon (mélange de glucose, d'oligomères et de polymères de glucose) ne pouvaient pas être utilisés tels quels.

La demande de brevet européen EP 207.676 enseigne qu'il est préféré des polymères de glucose formant des solutions limpides et incolores à 10 % dans l'eau, ayant un poids moléculaire moyen en poids (Mw) de 5.000 à 100.000 daltons et un poids moléculaire moyen en nombre (Mn) inférieur à 8.000 daltons.

De tels polymères de glucose comprennent aussi de façon préférée au moins 80 % de polymères du glucose dont le poids moléculaire est compris entre 5.000 et 50.000 daltons, peu ou pas de glucose ou de polymères du glucose de DP inférieur ou égal à 3 (poids moléculaire 504) et peu ou pas de polymères de glucose de poids moléculaire supérieur à 100.000 (DP voisin de 600).

En d'autres termes, les polymères de glucose préférés sont des polymères de glucose de faible indice de polymolécularité (valeur obtenue en calculant le rapport Mw/Mn).

On conçoit en effet aisément pour cette application que les monomères ou polymères de faible poids moléculaire traversent rapidement la paroi péritonéale et sont ainsi sans intérêt durable pour la création d'un gradient de pression osmotique, et que les polymères de très haut poids moléculaire, dénués de pouvoir osmotique, sont à éviter et même à proscrire puisque potentiellement dangereux s'il leur advenait de précipiter consécutivement à leur rétrogradation.

Les procédés proposés dans cette demande de brevet EP 207.676 pour obtenir ces polymères de glucose de faible indice de polymolécularité à partir d'hydrolysats d'amidon consistent :

- soit à effectuer une précipitation fractionnée d'une maltodextrine à l'aide d'un solvant miscible à l'eau,
- soit à effectuer une filtration moléculaire de cette même maltodextrine au travers de différentes membranes possédant un seuil de coupure ou d'exclusion adéquat.

Dans les deux cas, ces procédés visent à éliminer à la fois les polymères de très haut poids moléculaire et les monomères ou oligomères de faible poids moléculaire.

Ces procédés ne donnent toutefois pas satisfaction tant du point de vue de leur mise en œuvre que du point de vue des rendements et de la qualité des produits qu'ils permettent d'obtenir.

Soucieuse de mettre au point un procédé de fabrication d'un polymère de glucose complètement soluble dans l'eau et de faible indice de polymolécularité préférentiellement inférieur à 2,5, ayant de préférence un Mn inférieur à 8.000 daltons et possédant un Mw compris entre 12.000 et 20.000 daltons, procédé qui soit dépourvu des inconvénients de l'art antérieur, la société Demanderesse, s'est attachée à résoudre ce problème dans son brevet EP 667.356, en partant d'un amidon hydrolysé, plutôt que d'une maltodextrine.

Ce procédé consiste à :

- hydrolyser par voie acide un lait d'amidon cireux jusqu'à un D.E. compris entre 8 et 15 ;
- éventuellement compléter cette hydrolyse acide par une hydrolyse enzymatique à l'aide d'alpha-amylase bactérienne jusqu'à un D.E. compris entre 11 et 18 ;
- chromatographier sur résines cationiques fortes macroporeuses sous forme alcaline ou alcalino-terreuse cet hydrolysat double acide-enzyme ;
- collecter le polymère de glucose exclu lors de cette étape de chromatographie.

Dans ce brevet, pour obtenir un polymère de glucose présentant un indice de polymolécularité inférieur à 2,5, il est collecté le polymère de glucose exclu lors de cette étape de chromatographie dans un rendement pondéral de l'ordre de 60 % de l'hydrolysat d'amidon mis en œuvre en amont de l'étape de chromatographie.

Ce polymère de glucose contient alors de préférence moins de 3 % de glucose et de polymères de glucose de DP inférieur ou égal à 3 et moins de 0,5 % de polymères de glucose de DP supérieur à 600.

La demande WO2007/099212 décrit également un procédé de préparation de polymères de glucose adaptés à la dialyse péritonéale. Le procédé se termine par des étapes de fractionnement dans lesquelles les fractions de bas poids moléculaires sont éliminées, en particulier celles présentant une masse moléculaire inférieure à 9000, alors que les autres fractions, de haut poids moléculaire, sont récupérées. Plus précisément, le procédé comprend une étape de traitement par charbon actif (NORIT SX+), un fractionnement avec un seuil de coupure de 9000 daltons, une récupération du rétentat, puis une étape de déminéralisation et de traitement par charbon actif (NORIT SX+).

Il est finalement désormais admis par les experts du domaine de la dialyse péritonéale que ces polymères de glucose, utilisés pour leur pouvoir osmotique, donnent toute satisfaction.

Il est cependant à déplorer des risques de contamination microbienne de ces préparations destinées à la dialyse péritonéale.

Par exemple, le cathéter implanté dans la cavité péritonéale est une porte d'entrée propice aux germes. Les nombreuses manipulations sur le cathéter lors des phases d'infusion et de drainage augmentent le risque d'infection locale ou générale.

En outre, un facteur de risque supplémentaire de contamination peut être directement lié aux impuretés qui peuvent polluer les polymères de glucose utilisés comme agents osmotiques.

Il est en effet connu que les circuits de production des polymères de glucose peuvent être contaminés des microorganismes, ou par des substances pro-inflammatoires contenus dans lesdits microorganismes.

Il est par exemple décrit en amidonnerie la contamination des amidons de maïs ou de blé par des microorganismes de type levures, moisissures et bactéries, et plus particulièrement par des bactéries acidothermophiles de type *Alicyclobacillus acidocaldarius* (bactéries extrémophiles qui se développent dans les zones chaudes et acides du circuit).

Le risque majeur pour le patient qui reçoit ces produits contaminés est alors la péritonite.

Le soupçon clinique de la péritonite est diagnostiqué lors du développement d'un trouble dans le dialysat associé avec les manifestations cliniques variables que sont la douleur abdominale, la nausée, le vomissement, la diarrhée et la fièvre.

Ces épisodes de péritonite sont provoqués par des infections bactériennes intrapéritonéales, et le diagnostic est habituellement facilement établi par les cultures positives de dialysat.

La « péritonite stérile », également décrite en tant que péritonite aseptique, chimique, ou culture-négative, est quant à elle typiquement provoquée par un irritant chimique ou un corps étranger.

Depuis l'introduction de l'icodextrine pour la préparation de solutions de dialyse péritonéale, des cas isolés de péritonite aseptique ont été rapportés, pouvant être liés à des causes diverses et notamment l'induction par des substances pro-inflammatoires potentiellement présentes.

Or, ces substances proinflammatoires ne sont pas détectées par les tests habituellement effectués pour déterminer l'innocuité de telles préparations.

Les tests décrits aujourd'hui dans les Pharmacopées pour la détection de substances pyrogènes sont en effet les suivants :

- Le test dit LAL, de détection des endotoxines bactériennes (LPS), composants majoritaires des bactéries Gram négatives,
- Le test pyrogène lapin, de détection des endotoxines bactériennes (LPS) ainsi que les β glucanes, composants des parois de flores fongiques (levures et moisissures).

Bien que généralement fiables, ces deux tests présentent leurs limites.

Le test pyrogène lapin est fondé sur la détection indirecte de substances pyrogènes par la mesure d'une élévation de température du lapin auquel a été injecté le produit contenant ces substances (réponse fébrile).

Ce test peut donner lieu à des faux négatifs, si la substance indésirable présente une activité biologique trop faible ou une concentration trop basse pour induire une réponse systémique pyrogène.

Cependant, cette substance peut posséder une activité biologique ou concentration suffisante pour produire une réaction inflammatoire locale.

Les autres impuretés biologiques (ADN,...) ne sont pas détectées. Il en est de même pour les peptidoglycanes, composants majoritaires des membranes cellulaires des bactéries Gram positives.

La manifestation des péritonites aseptiques observées avec les solutions de dialyse péritonéale contenant de l'icodextrine témoigne donc, pour certains cas, de la façon dont certaines substances peuvent échapper aux tests décrits dans les pharmacopées et peuvent être à l'origine d'effets cliniques indésirables.

Pour remédier à cette situation, la société BAXTER a proposé de placer les efforts dans la détection des contaminants microbiens Gram positifs dans ses solutions pour dialyse péritonéales, ou directement sur les polymères de glucose purifiés destinés à rentrer dans la composition desdites solutions pour dialyse péritonéale.

En particulier, dans son brevet EP 1.720.999, la société BAXTER propose de développer une méthode basée sur la détection des peptidoglycanes.

Cette détection est alors préconisée directement sur les préparations pour dialyse péritonéale, ou sur les polymères de glucose purifiés.

Cette méthode consiste à réaliser sur les polymères de glucose :

- un essai de « biocharge » pour détecter le microorganisme Gram positif thermophile acidophile *Alicyclobacillus acidocaldarius,* puis
- une stérilisation desdits polymères de glucose, puis
- un test consistant à ajouter un réactif capable de réagir avec les peptidoglycanes pour induire une réaction en cascade de sérine-protéase,
- une quantification desdits peptidoglycanes.

En d'autres termes, la première étape détecte la présence de microorganismes vivants susceptibles d'avoir contaminé le circuit de purification des polymères de glucose, la seconde, par stérilisation, les élimine et la troisième détecte les peptidoglycanes issus des débris cellulaires non éliminés par l'étape précédente de stérilisation.

S'il est déterminé que la quantité de ces peptidoglycanes recherchée dans les polymères de glucose est bien inférieure à un certain seuil (10 ng/ml d'une solution à 7,5% de polymère de glucose, soit 133 ng/g de polymères de glucose), ces polymères de glucose sont alors utilisés pour préparer la solution de dialyse péritonéale proprement dite.

En d'autres termes, pour empêcher la survenue de ces épisodes de péritonites aseptiques, la société BAXTER, dans son brevet EP 1.720.999, propose d'assurer le contrôle de la fabrication et l'usage des solutions de dialyse péritonéale, à l'aide d'un protocole de détection de peptidoglycanes dans la solution de dialyse péritonéale.

La société BAXTER a également proposé pas moins de quatre autres procédés pour détecter ou éviter la présence de peptidoglycanes dans les composants destinés à la fabrication de solutions pour dialyse péritonéale, ou dans les solutions pour dialyse péritonéale en tant que telles :
Dans sa demande de brevet internationale WO 2009/117302, la société BAXTER décrit l'élimination des substances contaminantes par passage sur résine échangeuse d'ions.

Il est cependant à déplorer que cette technologie n'est que partiellement efficace, les inventeurs reconnaissant eux-mêmes que la finalité de cette adsorption sur résine est de « réduire » le risque de contamination, car elle ne retient qu'une « portion » des contaminants microbiens présents aussi bien dans les polymères de glucose que dans la solution finale pour dialyse péritonéale.

Il est même recommandé de passer les polymères de glucose ou la solution pour dialyse péritonéale sur plusieurs résines échangeuses d'ions en série, ce qui constitue un procédé de décontamination particulièrement lourd.

Un test de détection des peptidoglycanes est d'ailleurs mis en œuvre (analyse IL6) pour vérifier l'innocuité des solutions ainsi obtenues.

Dans sa demande de brevet internationale WO 2009/117303, il est proposé une méthode de détection des peptidoglycanes dans les polymères de glucose ou directement dans la préparation pour dialyse péritonéale comprenant la détermination de la réponse aux IL-6 établie en fonction d'une concentration en substance pro-inflammatoire prise comme référence, et l'établissement d'une courbe dose/réponse de la réponse aux IL6 en regard de diverses concentrations en substances pro-inflammatoires.

Cette méthode constitue donc un perfectionnement de la méthode de détection développée dans la demande de brevet EP 1.720.999.

Cette méthode nécessite cependant d'utiliser les cellules productrices d'IL6 isolées de sujets humains hypersensibles aux substances pro-inflammatoires particulières, en l'occurrence aux peptidoglycanes.

Une fois encore, cette méthode de diagnostic permet de rejeter les lots contaminés avec une grande sensibilité, mais ne permet pas de prévenir ladite contamination.

Dans sa demande brevet internationale WO 2009/117304, il s'agit de filtrer la solution de polymère de glucose ou la préparation pour dialyse péritonéale sur membrane d'ultrafiltration présentant un seuil de coupure de 30 kDa.

BAXTER aurait ainsi établi que la taille des contaminants comprise entre 30 et 100 kDa serait plus probablement celle qui cause les péritonites aseptiques, tandis que les contaminants de taille plus petite n'auraient aucun effet néfaste.

Le procédé décrit par BAXTER dans cette demande de brevet consiste donc à filtrer toute solution de polymère de glucose ou préparation destinée à la dialyse péritonéale sur cette membrane d'ultrafiltration et de tester, à l'aide du même test de détection que celui décrit dans la demande de brevet WO 2009/117303, le rétentat et le filtrat ainsi obtenu.

Le principe est le suivant : On élimine les solutions de polymère de glucose si, après ultrafiltration, le rétentat provoque une réaction pro-inflammatoire et que le filtrat n'en provoque pas.

On gardera par contre les solutions de polymère de glucose si le rétentat et le filtrat provoquent une réaction pro-inflammatoire et que la réponse du filtrat est supérieure à celle du rétentat.

Si le rétentat et le filtrat provoquent une réaction pro-inflammatoire et que la réponse du filtrat est inférieure à celle du rétentat, on examine la nature de la réponse du filtrat :

- si la réponse du filtrat est de 50 % et plus égale à celle du rétentat, on garde les solutions,

- si la réponse du filtrat est moins de 50 % de celle du rétentat, on rejette les solutions.

Cette méthode n'est cependant pas satisfaisante, car elle se base sur le postulat de la non innocuité des fragments de petite taille, et de ce fait, aucune technique n'est proposée pour les éliminer.

Dans sa demande de brevet 2009/117558, BAXTER agit par le biais d'enzymes de dégradation des composants des parois cellulaires des contaminants microbiens.

Ces enzymes, tel le lysozyme, utilisées sous forme soluble ou immobilisée, sont mises en œuvre seules ou en combinaison avec d'autres enzymes de dégradation des contaminants microbiens, et le polymère de glucose ou la préparation pour dialyse ainsi traités sont testés pour leur réponse aux cytokines.

Ce traitement enzymatique sur le produit final ou sur la préparation pour dialyse sera alors réellement prescrit aux patients si aucune réponse aux cytokines n'est détectée.

Cependant, une fois encore, rien n'est proposé pour éliminer les débris des composants des parois cellulaires ainsi hydrolysés, ni pour éliminer les substances qui contaminent naturellement les préparations enzymatiques utilisées.

De tout ce qui précède, il résulte qu'aucune solution technique pour sécuriser les polymères de glucose destinés à la préparation de solution pour dialyse péritonéale, ou pour sécuriser la solution pour dialyse péritonéale en tant que telle, n'est complètement satisfaisante.

Il est du mérite de la Société Demanderesse d'avoir trouvé que la solution doit reposer avant tout sur le contrôle de la qualité de la matière première mise en œuvre pour la préparation des polymères de glucose destinées à la préparation de solution pour dialyse péritonéale, i.e. de proposer un procédé simple et efficace d'élimination des contaminants des hydrolysats d'amidon en tant que tels.

En d'autres termes, il s'agit d'agir sur le problème de contamination à sa source, et pas seulement sur les produits finaux ou sur la solution finale pour dialyse péritonéale.

La présente invention se rapporte donc à un procédé de préparation de polymères de glucose destinés à la fabrication de solutions pour dialyse péritonéale, procédé qui comprend la décontamination des hydrolysats d'amidon à partir desquels seront préparés lesdits polymères de glucose.

Cette décontamination vise à garantir la production d'hydrolysats d'amidon ne devant pas présenter de contaminants résiduels, i.e. garantir des teneurs inférieures ou égales aux valeurs suivantes, i.e. :

- pour les microorganismes vivants : flore mésophile totale : < 50 /g ; moisissures et levures : < 15 /g ; Bacillus acidothermophiles : < 10 /g.
- pour les endotoxines (LPS) et $\beta$ glucanes, via le test LAL (méthode gel clot en point final) utilisant des réactifs fabriqués par CHARLES RIVER-ENDOSAFE (Lysat LAL de sensibilité 0,015 E.U/ml réf. OR15015 et endotoxines CSE 500 ng ou 10 ng par flacon réf.E110 ou E120) : $\leq$ 0.6 EU/g
- pour les peptidoglycanes et $\beta$-glucanes via un test de haute sensibilité mis au point par la société Demanderesse : < 8 ng/g de polymères de glucose.

Par « test de haute sensibilité mis au point et validé par la société Demanderesse », on entend un test développé et validé par la société Demanderesse, en adaptant le kit SLP-HS single set ref. 293-58301 fabriqué et commercialisé par la société WAKO Pure Chemical Industries Ltd.

Ce test consiste à utiliser le réactif dit « SLP-HS » (Silkworm Larvea Plasma-High sensitivity) réactif préparé à partir du plasma de ver à soie, capable de :

- réagir avec les peptidoglycanes et $\beta$-glucanes contenus dans une solution de polymère de glucose préparée à 5 % dans de l'eau (eau spéciale pour test LAL par exemple),
- induire une réaction en cascade de sérine-protéase et
- de détecter et/ou de quantifier lesdits peptidoglycanes et $\beta$-glucanes au moyen d'un lecteur de tubes TOXINOMETER fabriqué et commercialisé par la société WAKO Pure Chemical Industries Ltd à des seuils très faibles, i.e. :

  • une limite de détection (LD) à un seuil d'environ 0,05 ng/ml (soit 1 ng/g de polymère de glucose) et
  • une limite de quantification (LQ) à un seuil d'environ 0,15 ng/ml (3 ng/g de polymère de glucose).

(LD et LQ déterminées dans le produit polymère de glucose testé)
De manière plus précise, le test SLP-HP consiste à :

- préparer le polymère de glucose à tester en solution à 5 % dans de l'eau de qualité adéquate (eau spéciale pour test LAL par exemple),
- réaliser une gamme étalon de peptidoglycanes dans l'eau sur le domaine d'application de 0,04 à 2,5 ng/ml (valeurs cibles) avec le standard de peptidoglycanes (extrait de *Staphylococcus Aureus)* du kit SLP-HS single set pour l'établissement d'une droite d'étalonnage (régression linéaire en échelle logarithmique Ta = f(teneur en PG)),

- introduire 100 $\mu$l de la solution préparée à tester dans le tube HS-SLP après reconstitution par ajout de 100 $\mu$l du diluant (fourni dans le kit précédemment cité),
- introduire le tube SLP-HS dans le puit d'incubation du lecteur de tube TOXINOMETER (WAKO Pure Chemical Ltd) thermostaté à 30°C et paramétré selon les conditions prescrites par le fabricant, la teneur en PGs de la solution à tester étant calculée au moyen de la droite d'étalonnage établie.

Le résultat est exprimé en ng/ml de solution à 5 % testée puis en ng/g de polymère de glucose.

L'invention concerne un procédé de décontamination d'un hydrolysat d'amidon, pour la préparation de polymères de glucose destinés à la dialyse péritonéale, caractérisé en ce qu'il comprend les étapes suivantes :

1) préparer un hydrolysat d'amidon par hydrolyse enzymatique ou chimique d'amidon de manière à atteindre un équivalent Dextrose (DE) inférieur à 20, ou par hydrolyse par voie acide d'un lait d'amidon cireux jusqu'à un D.E. compris entre 8 et 15,
2) filtrer ledit hydrolysat d'amidon de manière à éliminer tout contaminant présentant la taille d'un microorganisme de type levures, moisissures ou bactéries, soit à l'aide d'une filtration membranaire de diamètre de pore de 0,22 $\mu$m, optionnellement précédé d'une préfiltration membranaire d'un diamètre de pore de 0,45 $\mu$m, soit à l'aide d'une ultrafiltration sur membrane présentant un seuil de coupure de 300.000 Da,
3) traiter ledit hydrolysat d'amidon ainsi débarrassé des microorganismes contaminants par une enzyme de dégradation des constituants polysaccharidiques des parois cellulaires, la laminarinase, la laminarinase étant introduite dans les hydrolysats d'amidon à la concentration de 0.001 ‰ à 1 % sur sec en poids d'hydrolysats d'amidon, et étant mise en œuvre à 10% de matière sèche à une température de 50°C, à un pH de 4,6, pendant 5 à 24 heures,
4) ultrafiltrer l'hydrolysat d'amidon ainsi traité enzymatiquement avec une membrane ayant un seuil de coupure de 20.000 Da à 50.000 Da,
5) traiter le perméat d'ultrafiltration contenant l'hydrolysat d'amidon ainsi obtenu sur charbon actif à haute capacité d'adsorption,
6) collecter l'hydrolysat d'amidon ainsi décontaminé.

Dans la première étape de ce procédé conforme à l'invention, l'hydrolysat d'amidon peut être préparé :

- soit par hydrolyse classique par voie enzymatique ou chimique de l'amidon de diverses sources végétales telles le blé, maïs, pomme de terre, pois, riz, manioc... de manière à atteindre un équivalent Dextrose (DE) inférieur à 20, caractéristique des maltodextrines,
- soit par hydrolyse par voie acide d'un lait d'amidon cireux jusqu'à un D.E. compris entre 8 et 15 éventuellement complétée par une hydrolyse enzymatique à l'aide d'alpha-amylase bactérienne jusqu'à un D.E. compris entre 11 et 18, selon l'enseignement du brevet EP 667.356 évoqué ci-dessus.

Pour tester la robustesse de son procédé de décontamination, comme il sera exemplifié ci-après, la société Demanderesse dispose d'hydrolysats d'amidon de maïs ou de maltodextrines contaminés artificiellement ou non :

- par les 4 catégories de contaminants présentés ci-dessus,
- par des peptidoglycanes (sous forme libre ou liée, i.e. à la surface de cellules vivantes) avec ou sans endotoxines,
- par des $\beta$-glucanes et des peptidoglycanes, avec ou sans endotoxines.

Dans la deuxième étape de ce procédé conforme à l'invention, il est procédé à l'élimination des microorganismes susceptibles de contaminer lesdits hydrolysats d'amidon, i.e. levures, moisissures et bactéries, et notamment les bactéries acidothermophiles de type *Alicyclobacillus acidocaldarius,* leur taille étant supérieure aux diamètres des pores de filtration.

Toute méthode connue en soi de l'homme du métier est utilisable, mais la société Demanderesse recommande de mettre en œuvre :

- soit une filtration stérilisante qui consiste principalement en une filtration membranaire de diamètre de pore de 0,22 $\mu$m, précédé le cas échéant d'une préfiltration membranaire d'un diamètre de pore de 0,45 $\mu$m.

La filtration est réalisée par plusieurs filtres à cartouches insérées dans un carter vertical vers lequel l'hydrolysat d'amidon est dirigé. Ces filtres à cartouches sont fournis par les sociétés PALL ou MILLIPORE. La taille des cartouches peut être de 10, 20 ou 30 pouces, et le nombre de cartouches installées permet d'obtenir une surface de filtration suffisante afin de passer un débit de produit entre 1 et 20 l/minutes/m². 

Ces filtres à cartouches ont des capacités de résistance pour un travail en continu à haute température, de l'ordre de 75°C et pour passer le débit précédemment cité durant une période supérieure de 700 h. Le fait de travailler à une température de 75°C permet de limiter tout développement microbiologique, notamment des flores thermophiles.

Leur résistance à la température permet également de réaliser une stérilisation avant leur mise en service. Cette stérilisation consiste à faire passer de la vapeur 2 bar à travers le carter pendant une période de 20 minutes. Cette stérilisation est suivie par un rinçage à l'eau purifiée pendant une période de 5 minutes.

Ces filtres possèdent également des capacités de résistance à certains produits chimiques utilisés pour le nettoyage des équipements, et notamment l'acide peracétique à une concentration de 5°/∞.

Un test d'intégrité est réalisable sur ces cartouches à l'aide d'un intégritest de la société MILLIPORE par exemple. Ce test d'intégrité est réalisé à la mise en place des cartouches afin d'en vérifier le montage. Ce test est ensuite réalisé avant chaque nettoyage des équipements et enfin avant le démontage afin de valider leur bon fonctionnement durant la phase de production.

Le delta de pression (ΔP) de travail de ces filtres ne doit pas dépasser les 2 bar afin de garantir leur intégrité. Si tel est le cas, ces filtres doivent être remplacés par de nouveaux.

- soit une ultrafiltration sur membrane présentant un seuil de coupure de 300.000 Da.

Le seuil de coupure est ainsi choisi de manière à retenir les contaminants cellulaires éventuels dans le rétentat.

Le seuil de coupure permet de retenir les microorganismes, i.e. levures, moisissures et bactéries et notamment les bactéries acidothermophiles de type *Alicyclobacillus acidocaldarius.*

La surface du filtre est déterminée en fonction de la nature du fluide et du débit à traiter.

Les membranes d'ultrafiltration peuvent être de type céramique ou organique. Ces deux types de membranes ayant une résistance différente à la température et aux produits chimiques, il sera préféré les membranes de type céramiques qui permettent de travailler à des températures supérieures à 75°C.

Leur résistance à la température permet de réaliser une stérilisation à la vapeur avant leur mise en service. Cette stérilisation consiste à faire passer de la vapeur 2 bar à travers le carter pendant une période de 20 minutes. Cette stérilisation est suivie par un rinçage à l'eau purifiée pendant une période de 20 minutes.

Il convient également de travailler à une température de l'ordre de 75°C pour éviter tout développement microbiologique.

Ces filtres possèdent également des capacités de résistance à certains produits chimiques utilisés pour le nettoyage des équipements, et notamment l'acide peracétique à une concentration de 5‰ et la soude à une concentration de 1%.

La pression de l'hydrolysat d'amidon en alimentation est comprise entre 2 et 20 bar et est régulée par la pompe alimentant ce module. Dans le cas où la pression maximale est atteinte mais que le débit de l'hydrolysat d'amidon est trop faible, il convient de réaliser un nettoyage à la soude des membranes afin qu'elles retrouvent une pleine efficacité.

Le suivi de l'abaissement du taux de contaminants peut être analysé en faisant des prélèvements périodiques sur le perméat.

Une élimination efficace des microorganismes sera considérée si les prélèvements sur le filtrat présentent un taux inférieur au seuil de quantification des méthodes classiques de dosage.

Dans la troisième étape de ce procédé conforme à l'invention, il est procédé au traitement dudit hydrolysat d'amidon ainsi débarrassé des microorganismes contaminants par une enzyme de dégradation des constituants polysaccharidiques des parois cellulaires, la laminarinase.

Après l'élimination des microorganismes vivants, cette étape permet donc d'éliminer les molécules toxiques résiduelles (libérés par les microorganismes ou encore présents dans les débris de membranes cellulaires).

Cette enzyme de dégradation des constituants polysaccharidiques des parois cellulaires est choisie de manière à rompre les membranes des deux germes contaminants majeurs des hydrolysats d'amidon (levures et bactéries Gram (+)) qui auraient pu échapper à l'étape de filtration précédente, et surtout pour hydrolyser leurs β-glucanes et peptidoglycanes qui peuvent se présenter sous forme libre dans lesdits hydrolysats d'amidon.

La laminarinase présente une activité endo-β-1,3 glucanase (EC 3.2.1.6), qui détruit la paroi cellulaire des levures et de certaines moisissures.

Le lysozyme, hydrolase acide (EC 3.2.1.17), détruit la paroi bactérienne des bactéries gram(+) en catalysant l'hydrolyse des glycosaminoglycanes la constituant. Il hydrolyse les liaisons covalentes entre l'acide N-acétyl-muramique avec le 4e atome de carbone du N-acétyl-glucosamine des peptidoglycanes. Le "squelette" du peptidoglycane de la paroi bactérienne est en effet un copolymère constitué de l'enchaînement covalent de ces deux molécules, en alternance, sur lesquelles sont fixés des peptides qui pontent les chaînes du polymère.

La laminarinase est introduite dans les hydrolysats d'amidon à la concentration de 0.001 ‰ à 1 % sur sec en poids d'hydrolysats d'amidon, de préférence compris entre 0,1 % à 0,5 %.

La laminarinase est mise en œuvre sur les hydrolysats d'amidon à 10% de matière sèche à une température de 50°C, à un pH de 4,6, pendant 5 à 24 heures, de préférence pendant 20 heures, comme il sera exemplifié ci-après.

Le lysozyme, peut quant à lui être mis en œuvre sur les hydrolysats d'amidon à 10% de matière sèche à une température de 37°C, à un pH de 7, pendant 5 à 24 heures, de préférence pendant 20 heures, comme il sera exemplifié ci-après.

Dans la quatrième étape de ce procédé conforme à l'invention, il est procédé à une ultrafiltration tangentielle de l'hydrolysat d'amidon ainsi traité enzymatiquement.

Le seuil de coupure de la membrane d'ultrafiltration est choisi de manière à retenir les éventuels produits de dégradation des β-glucanes et de peptidoglycanes dans le rétentat et permet surtout de retenir les enzymes mises en œuvre à l'étape précédente.

La membrane d'ultrafiltration présente alors un seuil de coupure compris entre 20.000 et 50.000 daltons, de préférence de l'ordre de 30.000 daltons.

Dans la cinquième étape de ce procédé conforme à l'invention, il est procédé à un passage de l'hydrolysat d'amidon ainsi obtenu, c'est-à-dire le perméat ou filtrat, sur charbon actif à haute capacité d'adsorption.

La société Demanderesse recommande de prendre un soin tout particulier à la mise en œuvre de cette étape de traitement au charbon actif, qui constitue une étape clef de cette première variante du procédé conforme à l'invention.

La société Demanderesse a en effet trouvé que le choix d'une qualité appropriée du charbon actif à utiliser dans cette étape de finition conditionne la quasi-absence des contaminants susceptibles d'être détectés dans les hydrolysats d'amidon ainsi traités (« quasi » s'entendant à des concentrations bien inférieures au seuil de quantification des méthodes classiques de dosage).

Il est connu de l'homme du métier que le charbon actif est un matériau carboné inerte adsorbant constitué d'un réseau poreux développant une surface considérable pouvant atteindre jusqu'à 1.500 m$^2$ par gramme de matière (les diamètres des pores variants entre 4 et 100.000 Angströms).

Pour être efficace, un charbon actif doit donc avoir une certaine surface interne développée au sein de la structure poreuse sur laquelle viendront s'adsorber les impuretés que l'on souhaite éliminer.

Pour modéliser cette capacité d'adsorption, l'homme du métier décrit habituellement le processus d'adsorption à l'aide d'une isotherme d'adsorption (isotherme de Langmuir, isotherme de Langmuir-Hinshelwood, isotherme BET ou isotherme de Freundlich).

L'isotherme d'adsorption est une courbe qui représente la quantité d'impuretés adsorbées par unité de masse de charbon activé en fonction de la concentration résiduelle des impuretés en solution.

Pour construire cette isotherme, on introduit des quantités connues et croissantes de charbon actif dans un volume de solution à traiter, et après un temps de contact donné, on mesure la concentration résiduelle d'impuretés adsorbées.

La société Demanderesse recommande donc, en préambule de toute mise en œuvre d'un charbon actif destiné ici à terminer la décontamination des hydrolysats d'amidon destinés à la préparation de polymères de glucose destinés à la dialyse péritonéale (cinquième étape de finition), de choisir précisément la qualité du charbon actif en modélisant son isotherme d'adsorption.

Il est ainsi réalisé un essai préliminaire pour choisir le charbon actif le plus efficace, essai qui consiste à tracer l'isotherme d'adsorption de Freundlich pour différentes qualités de charbon actif disponibles dans le commerce (voire même de tester différents lots d'un même type de charbon actif), en regard de leur capacité d'adsorption de quantités connues de contaminants de type endotoxines bactériennes et peptidoglycanes qui sont pris ici comme références.

Il s'agit donc de réaliser une qualification précise de tous les lots destinés à être utilisés dans cette étape clef de finition des hydrolysats d'amidon.

La société Demanderesse recommande de tester plus particulièrement ici la qualité de type « méso », i.e. présentant un indice de bleu de méthylène élevé ou la qualité de type « micro », i.e. présentant un indice d'iode élevé.

Ainsi, la méthode peut comprendre une étape préalable de détermination de l'isotherme d'adsorption de plusieurs charbons actifs, en particulier un de type « méso » et un de type « micro », et de sélectionner le charbon actif le plus approprié.

L'isotherme d'adsorption qui permet classiquement de déterminer la surface spécifique d'un charbon actif donné va donc être ici mise à contribution pour aider au choix du charbon actif qui permettra :

- d'adsorber tous les produits de dégradation de β-glucanes et de peptidoglycanes générés aux étapes précédentes, quelle que soit leur taille,
- et surtout d'adsorber les endotoxines susceptibles de contaminer les hydrolysats d'amidon, et également les endotoxines apportées par les enzymes en œuvre (aucune préparation industrielle commercialisée de ces enzymes n'en est malheureusement dépourvue).

Comme il sera exemplifié ci-après, le mode opératoire de la détermination de l'isotherme d'adsorption de Freundlich recommandé par la société Demanderesse consiste à mélanger l'hydrolysat d'amidon entrant avec chaque lot de charbon actif (5 doses croissantes comprises entre 0,125 % à 2 % sur la matière sèche à traiter, i.e. 0,125 % ; 0,25 % ; 0,5 % ; 1% et 2 %) à une température de 75°C pendant une heure.

La température de 75°C est la température de mise en œuvre classique des charbons actifs du commerce, validée pour la cinétique d'adsorption.

La durée d'une heure de temps de contact a été déterminée de manière à s'affranchir de la contrainte cinétique par rapport à la thermodynamique du système.

Quant au pH, il est fixé à une valeur de 4,5, car la société Demanderesse a déterminé que pour assurer une adsorption optimale des impuretés peptidoglycanes et endotoxines, il fallait préalablement conditionner l'échantillon d'hydrolysat d'amidon à une valeur de pH acide, i.e. de l'ordre 4,5.

Comme il sera exemplifié ci-après, la qualité du charbon actif pour cette cinquième étape de finition a été identifiée comme un charbon actif de type « micro », d'indice d'iode élevé.

Un charbon actif de type NORIT® SX +, commercialisé par la société NORIT, est tout à fait adapté à cette qualité.

Dans la dernière étape de ce procédé conforme à l'invention, l'hydrolysat d'amidon obtenu est alors collecté, et son contenu en contaminants analysé.

L'invention sera mieux comprise à l'aide des exemples qui suivent, lesquels se veulent illustratifs et non limitatifs.

Exemple de référence

**1** : **Préparation de l'hydrolysat d'amidon conformément à l'enseignement du brevet EP 667.356**

La matière première pour l'obtention des polymères de glucose selon l'invention est produite à partir d'amidon de maïs cireux de la manière suivante :

- nettoyage du maïs de manière à garder exclusivement les grains de maïs entier,
- trempe du maïs ainsi nettoyé en présence d'acide lactique de manière à assouplir les grains,
- broyage humide, puis séparation des différents constituants, i.e. germe, enveloppe cellulosique, protéines et amidon,
- nettoyage de l'amidon à contre courant avec de l'eau désinfectée de manière à purifier l'amidon aussi bien physico-chimiquement que bactériologiquement,
- centrifugation et séchage de l'amidon,
- mise en suspension de l'amidon dans une eau désinfectée à une matière sèche finale de 40 % et à une Température de 45°C à 50°C,
- acidification de la suspension d'amidon par addition d'HCl à un pH < 2, et accroissement de la température jusqu'à 115 à 120°C pendant 6 à 8 minutes,
- floculation des protéines et des matières grasses à ce pH,
- neutralisation de la suspension à pH 5,
- filtration de la suspension sur terre de diatomées (de manière à retenir les protéines, les matières grasses et la cellulose résiduelles),
- déminéralisation sur résine cationique forte et résine anionique faible,
- décoloration sur charbon actif standard,
- atomisation de la solution concentrée dans un atomiseur de type MSD commercialisée par la société NIRO.

Des lots particulièrement contaminés référencés « A-5250 » et « B-3063 » ont été choisis de manière distincte afin de tester la validité des procédés conformes à l'invention (plus ou moins difficiles à décontaminer en fonction de la nature et de la taille de ces différents contaminants peptidoglycanes, β-glucanes et endotoxines).

Le tableau I suivant présente la nature des contaminants identifiés dans ces différents lots.

A titre comparatif, est présentée également la nature des contaminants qu'il est possible de trouver dans six lots de maltodextrines commerciales « C ».

Tableau I.

| Hydrolysats d'amidon | *Alicyclo bacillus acidocaldarius* (Nombre de germes sur 1 g) | Levures (Nombre de germes sur 1 g) | Endotoxines + β-glucanes (EU/g) | Peptidoglycanes (ng/g) | Répartition β-glucanes / Endotoxines (%) |
|---|---|---|---|---|---|
| Méthodes de mesures | | | Test LAL | Test Haute sensibilité | Test LAL modifié |
| lot « A-5250 » n°1 | 0 | < 5 | 9, 6 | 2.462 | 90 / 10 |
| lot « A-5250 » n°2 | 0 | < 5 | 19,2 | 2.576 | 90 / 10 |
| lot « A-5250 » n°3 | 0 | < 5 | 9, 6 | 1.882 | 90 / 10 |
| lot « A-5250 » n°4 | 0 | < 5 | 4, 8 | 3.166 | 90 / 10 |
| lot « A-5250 » n°5 | 0 | < 5 | 9, 6 | 2.277 | 90 / 10 |
| lot « A-5250 » n°6 | 0 | < 5 | 9, 6 | 3.540 | 90 / 10 |
| Lot « B-3063 » n°1 | 150 | < 5 | 2,4 | 36.029 | 0 / 100 |
| Lot « B-3063 » n°2 | 150 | < 5 | 2,4 | 25.029 | 0 / 100 |
| Lot « B-3063 » n°3 | 150 | < 5 | 9, 6 | 27.260 | 0 / 100 |
| Lot « B-3063 » n°4 | 150 | < 5 | 1,2 | 10.241 | 0 / 100 |
| Lot « B-3063 » n°5 | 150 | < 5 | 2,4 | 55.691 | 0 / 100 |
| Lot « B-3063 » n°6 | 160 | < 5 | 2,4 | 46.367 | 0 / 100 |
| Lot « C » n°1 | 2 | < 5 | 9, 6 | 13.660 | 75 / 25 |
| Lot « C » n°2 | 2 | < 5 | 3, 6 | 9.374 | 75 / 25 |
| Lot « C » n°3 | 2 | < 5 | 9, 6 | 10.556 | 75 / 25 |
| Lot « C » n°4 | 2 | < 5 | 4, 8 | 9.353 | 75 / 25 |
| Lot « C » n°5 | 2 | < 5 | 9, 6 | 11.920 | 75 / 25 |
| Lot « C » n°6 | 2 | < 5 | 9, 6 | 16.288 | 75 / 25 |

Ces différents lots offrent donc trois options de contamination possible :

-   6 lots « A-5250 » contaminés essentiellement par des peptidoglycanes (polysaccharides sous forme libre) et des β glucanes,

-   6 lots « B-3063 » contaminés essentiellement par des peptidoglycanes (cellules vivantes ou polysaccharides sous forme libre) et des endotoxines, sans trace de β glucanes,

-   6 lots « C » dont le profil de contamination est à large spectre, i.e. contenant toutes les catégories de contaminants.

Exemple de référence 2 : Choix des qualités de charbon actif en fonction de la nature et de la quantité des contaminants β-glucanes et peptidoglycanes susceptibles d'être retrouvés dans les hydrolysats d'amidon des lots A-5250 et B-3063 et d'une maltodextrine commerciale C

Comme indiqué ci-avant, les étapes de traitement au charbon actif sont surtout mises en œuvre pour adsorber les débris cellulaires de type endotoxines, β-glucanes et peptidoglycanes.
Le choix de la qualité de charbon actif repose sur l'analyse des isothermes de Freundlich tracés pour chaque charbon actif directement sur l'hydrolysat d'amidon à décontaminer.
Comme il va être démontré ci-dessous, le choix du charbon actif dépendra directement de la charge en contaminants (en nature et quantité) présents dans les hydrolysats d'amidon à traiter.
Comme indiqué ci-avant, le mode opératoire de la détermination de l'isotherme d'adsorption de Freundlich consiste à mélanger l'hydrolysat d'amidon (à 10 % de matière sèche) avec chaque type de charbon actif (5 doses croissantes comprises entre 0,125 % à 2 % sur la matière sèche à traiter, i.e. 0,125 % ; 0,25 % ; 0,5 % ; 1% et 2 %) à une température de 75°C pendant une heure à un pH de 4,5.

On détermine ensuite l'efficacité relative des charbons actifs de type « méso » et « micro » en fonction de la nature des contaminants de chaque lot par la mesure de la quantité d'impuretés adsorbées par unité de masse de charbon activé en fonction de la concentration résiduelle (après une heure de réaction) des impuretés en solution.

**2.1 Détermination des isothermes de Freundlich des charbons actifs de type NORIT SX + et ENO-PC versus deux lots A-5250**

Tous les lots A présentent un profil semblable : contaminés essentiellement par des peptidoglycanes (polysaccharides sous forme libre) et des β glucanes.

Le tableau II suivant présente, pour 5 doses de charbon actif croissantes, les taux résiduels en β glucanes et peptidoglycanes résiduels des lots A-5250 n°1 et n°2 de l'exemple 1, après traitement avec les deux qualités de charbon actif.

Tableau II.

| Dose de charbon actif | Lot n°1 NORIT SX + | | Lot n°2 ENO-PC | |
| --- | --- | --- | --- | --- |
| | Méthode de détection LAL (EU/g) | Test Haute sensibilité peptidoglycanes (ng/g) | Méthode de détection LAL (EU/g) | Test Haute sensibilité peptidoglycanes (ng/g) |
| 0 | 9, 6 | 2.462 | 19,2 | 2.576 |
| 0,125 | 1,2 | 555 | 0,3 | 416 |
| 0,25 | 0,3 | 127 | 0,3 | 156 |
| 0,5 | 0,3 | 10 | < 0,3 | 113 |
| 1 | 0,3 | < 1 | < 0,3 | 13 |
| 2 | < 0,3 | < 1 | < 0,3 | < 1 |

Si l'on analyse les courbes d'adsorption pour les peptidoglycanes (responsables des péritonites aseptiques) pris ici comme référence, on constate que :
La courbe d'adsorption du charbon actif NORIT SX +, traçant

$$y = \frac{(Co - C)}{(dose\ de\ noir)} = f(C)$$

*où*

*Co = dose de contaminants initiale*
*C = dose de contaminants résiduels*
se traduit par l'équation mathématique :

$y = 2588\ X^{0,2771}$ avec un coefficient de corrélation $r^2$ de 0,9914.

La courbe d'adsorption du ENO-PC est quant à elle traduite par l'équation :

$$Y = 1045\ .\ x^{0,4156}$$

avec un $r^2$ (coefficient de corrélation) de 0,9190.

La linéarité de ces deux courbes prises séparément traduit bien l'efficacité d'adsorption des peptidoglycanes par ces deux qualités de charbon actif.

Mais en comparant les deux équations, il apparaît que :

- pour une dose « x » de peptidoglycanes par exemple de 2000 ng/g, l'efficacité relative de la qualité des charbons actifs NORIT/ENO-PC est de 86 % ; tandis que

- pour une dose « x » de peptidoglycanes par exemple de 10 ng/g, l'efficacité relative de la qualité des charbons actifs NORIT/ENO-PC est de 180 %.

Il s'en déduit que la qualité ENO-PC « mésopore » est bien adaptée à l'adsorption de grandes quantités de contaminants de peptidoglycanes, et que la qualité NORIT SX + « micropore » est bien adaptée à l'adsorption de petites quantités de peptidoglycanes résiduels.

Ce résultat permet, pour le lot A-5250, de définir l'ordre de mise en œuvre des charbons actifs dans les étapes de décontamination de ce lot particulier d'hydrolysat d'amidon : une étape de traitement au charbon actif ENO-PC suivi d'une étape de traitement au charbon actif NORIT SX +.

### 2.2 Détermination des isothermes de Freundlich des charbons actifs de type NORIT SX + et ENO-PC versus deux lots B-3063

Tous les lots B présentent un profil semblable : contaminé essentiellement par des peptidoglycanes et des endotoxines.

Le tableau III suivant présente, pour 5 doses de charbon actif croissantes, les taux résiduels en β glucanes et peptidoglycanes résiduels du lot B-3063 n°1 et n°2 de l'exemple 1, après traitement avec les deux qualités de charbon actif.

Tableau III.

| Dose de charbon actif | Lot n°1 NORIT SX + | | Lot n°2 ENO-PC | |
|---|---|---|---|---|
| | Méthode de détection LAL (EU/g) | Test Haute sensibilité peptidoglycanes (ng/g) | Méthode de détection LAL (EU/g) | Test Haute sensibilité peptidoglycanes (ng/g) |
| 0 | 2,4 | 36.029 | 2,4 | 25.029 |
| 0,125 | 0,5 | 140 | 0,3 | 928 |
| 0,25 | < 0,3 | 50 | < 0,3 | 300 |
| 0,5 | < 0,3 | 10 | < 0,3 | 83 |
| 1 | < 0,3 | < 1 | < 0,3 | 10 |
| 2 | < 0,3 | < 1 | < 0,3 | 5 |

Si l'on analyse les courbes d'adsorption pour les peptidoglycanes (responsables des péritonites aseptiques), on constate que :

La courbe d'adsorption du charbon actif NORIT SX + se traduit par l'équation mathématique :

$y = 32543\ x^{0,4088}$ avec un coefficient de corrélation $r^2$ de 0,9711.

La courbe d'adsorption du ENO-PC est quant à elle traduite par l'équation :

$$Y = 6543 \cdot x^{0,4849}$$

avec un $r^2$ (coefficient de corrélation) de 0,9813.

La linéarité de ces deux courbes prises séparément traduit bien l'efficacité d'adsorption des peptidoglycanes par ces deux qualités de charbon actif.

Mais en comparant les deux équations, il apparaît que :

- pour une dose « x » de peptidoglycanes par exemple de 2000 ng/g, l'efficacité relative de la qualité des charbons actifs NORIT/ENO-PC est de 279 % ; tandis que
- pour une dose « x » de peptidoglycanes par exemple de 10 ng/g, l'efficacité relative de la qualité des charbons actifs NORIT/ENO-PC est de 417 %.

Il s'en déduit que la qualité ENO-PC « mésopore » n'est pas adaptée à l'adsorption des peptidoglycanes de ce lot B-3063, et que la qualité NORIT SX + « micropore » peut être utilisée ici pour l'adsorption de petites et grandes quantités de peptidoglycanes.

Ce résultat permet, pour le lot B-3063, de définir l'ordre de mise en œuvre des charbons actifs dans les étapes de décontamination de ce lot particulier d'hydrolysat d'amidon : ici deux étapes de charbon actif de type NORIT SX + en série.

**2.3 Détermination des isothermes de Freundlich des charbons actifs de type NORIT SX + et ENO-PC versus deux lots C**

Le tableau IV suivant présente, pour 5 doses de charbon actif croissantes, les taux résiduels en β glucanes/endotoxines et peptidoglycanes résiduels des lots C n°1 et n°2 de l'exemple 1, après traitement avec les deux qualités de charbon actif.

Tableau IV

| Dose de charbon actif | Lot n°1 NORIT SX + | | Lot n°2 ENO-PC | |
|---|---|---|---|---|
| | Méthode de détection LAL (EU/g) | Test Haute sensibilité peptidoglycanes (ng/g) | Méthode de détection LAL (EU/g) | Test Haute sensibilité peptidoglycanes (ng/g) |
| 0 | 9, 6 | 13.660 | 9, 6 | 9.374 |
| 0,125 | 1,2 | 775 | 1,2 | 896 |
| 0,25 | < 0,3 | 190 | 0, 6 | 369 |
| 0,5 | < 0,3 | 25 | < 0,3 | 106 |
| 1 | < 0,3 | 10 | < 0,3 | 26 |
| 2 | < 0,3 | < 1 | < 0,3 | 28 |

Si l'on analyse les courbes d'adsorption pour les peptidoglycanes (responsables des péritonites aseptiques), on constate que :

La courbe d'adsorption du charbon actif NORIT SX + se traduit par l'équation mathématique :

$y = 6377\ x^{0,4132}$ avec un coefficient de corrélation $r^2$ de 0,9874.

La courbe d'adsorption du ENO-PC est quant à elle traduite par l'équation :

$$Y = 783,97 \cdot x^{0,6559}$$

avec un $r^2$ (coefficient de corrélation) de 0,9353.

La linéarité de ces deux courbes prises séparément traduit bien l'efficacité d'adsorption des peptidoglycanes par ces deux qualités de charbon actif.

Mais en comparant les deux équations, il apparaît que :

-    pour une dose « x » de peptidoglycanes par exemple de 2000 ng/g, l'efficacité relative de la qualité des charbons actifs NORIT/ENO-PC est de 128 % ; tandis que
-    pour une dose « x » de peptidoglycanes par exemple de 10 ng/g, l'efficacité relative de la qualité des charbons actifs NORIT/ENO-PC est de 2349 %.

Il s'en déduit que la qualité ENO-PC « mésopore » n'est pas adaptée à l'adsorption des peptidoglycanes de ces lots C, et que la qualité NORIT SX + « micropore » peut-être utilisée ici pour l'adsorption de petites et grandes quantités de peptidoglycanes.

**Exemple 3 : Procédé de décontamination de l'hydrolysat d'amidon mettant en œuvre le** lysozyme (référence) ou la laminarase (invention), **suivi d'une étape d'ultrafiltration puis de charbon actif**

On choisit ici les lots A-5250 n°3 et n°5, les lots B-3063 n°3 et n°5, et les lots de maltodextrines commerciales C n° 3 et n°5.

S'il est relativement aisé d'éliminer les microorganismes de type *Alicyclobacillus acidocaldarius* et levures sur module de microfiltration 0,2 μm ou sur module d'ultrafiltration de seuil de coupure 300 kDa dans les conditions explicitées ci-avant, l'élimination des endotoxines, β-glucanes et/ou peptidoglycanes nécessite la mise en œuvre de combinaison d'étapes de traitement toutes particulières.

Comme il a été décrit ci-avant, cette combinaison d'étapes consiste :

- par l'utilisation d'enzyme spécifique de type lysozyme ou laminarinase, de dégrader les endotoxines, β-glucanes et peptidoglycanes, afin d'en réduire significativement la taille,
- par l'étape d'ultrafiltration, d'éliminer la fraction enzymatique et de retenir les impuretés amenées par les enzymes industrielles elles-mêmes,
- par l'étape finale de traitement au charbon actif, d'adsorber tous les débris de petite taille résiduels.

En ce qui concerne cette dernière étape de traitement au charbon actif, les courbes d'isotherme de Freundlich telles qu'établies dans l'exemple 2 enseignent que la qualité NORIT SX + est celle à mettre en œuvre.

Le tableau V suivant présente la mesure des contaminants β-glucanes et peptidoglycanes résiduels après traitements des lots A-5250 :

- au lysozyme (enzyme commercialisée par la société FUKA, présentant une activité de 70.000 U/mg), ou
- à la laminarinase (enzyme commercialisé par la société SIGMA sous le nom de marque CYTOHELICASE®, présentant une activité de 1 U/mg).

Il est important de noter que ces préparations enzymatiques ne sont pas exemptes de contaminants.

Il est ainsi déterminé que :

- le lysozyme FUKA présente un niveau d'endotoxines > 9,6 EU/ml et un niveau de petidoglycanes de 31000 ng/g
- la CYTOHELICASE® de SIGMA présente quant à elle un niveau d'endotoxines > 9,6 EU/ml et un niveau de petidoglycanes de 38000 ng/g.

Tableau V.

| Méthodes de mesures | Endotoxines + β-glucanes (EU/g) | Peptidoglycanes (ng/g) |
|---|---|---|
| | Test LAL | Test de haute sensibilité& peptidoglycanes |
| lot « A-5250 » n°3 | 9, 6 | 1.882 |
| 1) lysozyme (dose de 0,1 %) 20 h d'incubation | 9, 6 | 1.694 |
| 2) Traitement UF 30 kDa Facteur de Concentration Volumique = 10 perméat | 9, 6 | 639 |
| 3) Traitement au charbon actif NORIT SX + à 0,5 % Sur le perméat d'ultrafiltration | < 0,3 | 217 |
| lot « A-5250 » n°5 | 9,6 | 2.277 |
| 1) CYTOHELICASE® dose de 0,1 ‰ Après 20 h d'incubation | 38,4 | 191 |
| 2) Traitement UF 30 kDa Facteur de Concentration Volumique = 10 perméat | 0,6 | < 0,3 |
| 3) Traitement au charbon actif NORIT SX + à 0,5 % Sur le perméat d'ultrafiltration | **< 0,3** | < 20 |

Les résultats montrent que pour le lot A-5250 qui sera récupéré dans le perméat d'ultrafiltration traité au charbon actif, le traitement préalable à la laminarinase est bien plus efficace que celui au lysozyme pour éliminer les β-glucanes et peptidoglycanes, au sens où le traitement au lysozyme n'a même aucune action sur le lot A-5250.

Le tableau VI suivant présente la mesure des contaminants endotoxines et peptidoglycanes résiduels après traitements des lots B-3063.

EP 2 635 608 B1

Tableau VI

| Méthodes de mesures | Endotoxines + β-glucanes (EU/g) Test LAL | Peptidoglycanes (ng/g) Test de haute sensibilité& peptidoglycanes |
|---|---|---|
| Lot « B-3063 » n°3 | 9,6 | 27.260 |
| 1) lysozyme (dose de 0,1 %) 20 h d'incubation | 2,4 | 4.467 |
| 2) Traitement UF 30 kDa Facteur de Concentration Volumique = 10 perméat | 1,2 | 30 |
| 3) Traitement au charbon actif NORIT SX + à **0,5** % Sur le perméat d'ultrafiltration | **< 0,3** | **< 20** |
| lot « B-3063 » n°5 | 2,4 | 55.691 |
| 1) CYTOHELICASE® dose de 0,1 ‰ Après 20 h d'incubation | 4, 8 | 185 |
| 2) Traitement UF 30 kDa Facteur de Concentration Volumique = 10 perméat | < 0,3 | < 20 |
| 3) Traitement au charbon actif NORIT SX + à 0,5 % Sur le perméat d'ultrafiltration | **< 0,3** | **< 20** |

Les résultats montrent que pour le lot B-3063 qui sera récupéré dans le perméat d'ultrafiltration traité au charbon actif, le traitement préalable à la laminarinase est bien plus efficace que celui au lysozyme pour éliminer les peptidoglycanes.

Le tableau VII suivant présente la mesure des contaminants β-glucanes/endotoxines et peptidoglycanes résiduels après traitements des lots C.

Tableau VII

| Méthodes de mesures | Endotoxines + β-glucanes (EU/g) Test LAL | Peptidoglycanes (ng/g) Test de haute sensibilité& peptidoglycanes |
|---|---|---|
| lot « C » n°3 | 9, 6 | 10.556 |
| 1) lysozyme (dose de 0,1 %) 20 h d'incubation | 9, 6 | 17.968 |
| 2) Traitement UF 30 kDa Facteur de Concentration Volumique = 10 perméat | 9, 6 | 534 |
| 3) Traitement au charbon actif NORIT SX + à 0,5 % Sur le perméat d'ultrafiltration | **< 0,3** | **20** |
| lot « C » n°5 | 9, 6 | 11.920 |
| 1) CYTOHELICASE® dose de 0,1 ‰ Après 20 h d'incubation | 4,8 | 1293 |
| 2) Traitement UF 30 kDa Facteur de Concentration Volumique = 10 perméat | < 0,3 | 76 |
| 3) Traitement au charbon actif NORIT SX + à 0,5 % Sur le perméat d'ultrafiltration | **< 0,3** | **< 20** |

Les résultats montrent que pour le lot C qui sera récupéré dans le perméat d'ultrafiltration traité au charbon actif, le traitement préalable à la laminarinase est bien plus efficace que celui au lysozyme pour éliminer les endotoxines/β-glucanes et peptidoglycanes, au sens où le traitement au lysozyme n'a même aucune action sur le lot A-5250.

Ces résultats démontrent que le choix de l'enzyme de dégradation des parois cellulaires n'est ici pas anodin et est bien fonction de la nature des contaminants présents dans ces différents lots.

Le traitement à la CYTOHELICASE® sera clairement à préférer à celui par le lysozyme.

16

Exemple de référence **4 : Traitement par ultrafiltration puis charbon actif**

Comme il va être démontré, dans certains cas de figure, le traitement par ultrafiltration seule peut permettre l'abattement des endotoxines, β-glucanes et peptidoglycanes tout à fait satisfaisant.

La conduite du procédé est la même que celle décrite dans l'exemple 3, mais sans utilisation d'enzymes (donc sans modification du pH et de la température).

### 4.1 Traitement sur un lot A-5250

Le tableau VIII présente la teneur en contaminants résiduels β-glucanes et peptidoglycanes après chaque étape, pour le lot A-5250 n°4.

Tableau VIII.

| | Endotoxines + β-glucanes (EU/g) | Peptidoglycanes (ng/g) |
|---|---|---|
| Méthodes de mesures | Test LAL | Test de haute sensibilité& peptidoglycanes |
| Lot « A-5250 » n°4 | 4,8 | 3.166 |
| 1) Traitement UF 30 kDa Facteur de Concentration Volumique = 10 perméat | 4,8 | 883 |
| 2) Traitement au charbon actif NORIT SX + à 0,5 % Sur le perméat d'ultrafiltration | **< 0,3** | **< 20** |

La combinaison des étapes ultrafiltration et charbon actif de finition permet de garantir une décontamination des hydrolysats d'amidon du lot A-5250.

Si l'on compare aux valeurs du procédé du Tableau V (hors valeurs obtenues par le traitement au lysozyme) on note cependant que l'abattement en β-glucanes et peptidoglycanes est meilleur si le traitement à la CYTOHELICASE® est réalisé avant l'étape d'ultrafiltration, même si, après le traitement de finition au charbon actif NORIT SX +, les résultats sont identiques.

Ce traitement préalable à la CYTOHELICASE® est donc à préconiser pour optimiser l'efficacité du traitement de décontamination (capabilité du procédé de décontamination conforme à l'invention, i.e. son aptitude à produire de manière précise et répétable des hydrolysats d'amidon débarrassés de ses contaminants).

### 4.2 Traitement sur un lot B-3063

Le tableau IX présente la teneur en contaminants résiduels endotoxines et peptidoglycanes après chaque étape, pour le lot B-3063 n°4.

Tableau IX.

| | Endotoxines + β-glucanes (EU/g) | Peptidoglycanes (ng/g) |
|---|---|---|
| Méthodes de mesures | Test LAL | Test de haute sensibilité& peptidoglycanes |
| Lot « B-3063 » n°4 | 1,2 | 10.241 |
| 1) Traitement UF 30 kDa Facteur de Concentration Volumique = 10 perméat | 1,2 | < 20 |
| 2) Traitement au charbon actif NORIT SX + à 0,5 % Sur le perméat d'ultrafiltration | **< 0,3** | **< 20** |

La combinaison des étapes ultrafiltration et charbon actif de finition permettent de garantir une décontamination des hydrolysats d'amidon du lot B-3063.

Si l'on compare aux valeurs su procédé du Tableau VI (hors valeurs obtenues par le traitement au lysozyme) on note cependant que l'abattement en endotoxines et peptidoglycanes est également meilleur si le traitement à la CYTOHELICASE® est réalisé avant l'étape d'ultrafiltration, même si, après le traitement de finition au charbon actif NORIT SX +, les résultats sont identiques.

4.3 **Traitement sur un lot** C

Le tableau X présente la teneur en contaminants résiduels endotoxines et peptidoglycanes après chaque étape, pour le lot C n°4.

Tableau X.

| | Endotoxines + β-glucanes (EU/g) | Peptidoglycanes (ng/g) |
|---|---|---|
| Méthodes de mesures | Test LAL | Test de haute sensibilité& peptidoglycanes |
| Lot C n°4 | 4, 8 | 9.353 |
| 1) Traitement UF 30 kDa Facteur de Concentration Volumique = 10 perméat | 38, 4 | 340 |
| 2) Traitement au charbon actif NORIT SX + à **0,5** % Sur le perméat d'ultrafiltration | **< 0,3** | **< 20** |

La combinaison des étapes ultrafiltration et charbon actif de finition permettent de garantir une décontamination des hydrolysats d'amidon du lot C.
Si l'on compare aux valeurs su procédé du Tableau VII (hors valeurs obtenues par le traitement au lysozyme) on note cependant que l'abattement en endotoxines/β-glucanes et peptidoglycanes est également meilleur si le traitement à la CYTOHELICASE® est réalisé avant l'étape d'ultrafiltration, même si, après le traitement de finition au charbon actif NORIT SX +, les résultats sont identiques.

**Exemple 5 : Procédé de décontamination de l'hydrolysat d'amidon mettant en œuvre une succession d'étapes de charbon actif.**

Les lots « A-5250 » n°6 ; « B-3063 » n°6 et « C » n°6 débarrassés de leurs germes contaminants de la manière telle qu'exposée dans l'exemple 3 sont soumis aux traitements suivants.

**5.1. Deux traitements en série avec un charbon actif de même qualité.**

Il s'agit d'appliquer un double traitement avec le même charbon actif, en l'occurrence ici du NORIT SX + à 0,25 % sur sec caractéristique d'un traitement de type « micropore ».
Le tableau XI suivant présente les résultats obtenus. En témoin, un traitement unique au charbon actif avec double dose de NORIT SX + est également réalisé.

Tableau XI

| Hydrolysat d'amidon | | Endotoxines + β-glucanes (EU/g) | Peptidoglycanes (ng/g) |
|---|---|---|---|
| lot « A-5250 » n°6 | départ | 9, 6 | 3.540 |
| | 1er TN | 2,4 | 593 |
| | 2ème TN | < 0,3 | < 10 |
| | TN « double dose » | 0 | 60 |
| Lot « B-3063 » n°6 | départ | 2,4 | 46.367 |
| | 1er TN | < 0,3 | 223 |
| | 2ème TN | < 0,3 | < 10 |
| | TN « double dose » | < 0,3 | 76 |

(suite)

| Hydrolysat d'amidon | | Endotoxines + β-glucanes (EU/g) | Peptidoglycanes (ng/g) |
|---|---|---|---|
| Lot « C » n°6 | départ | 9, 6 | 16.288 |
| | 1er TN<br>2ème TN | < 0,3<br>< 0,3 | 80<br>< 10 |
| | TN « double dose » | < 0,3 | 14 |

Il apparaît clairement qu'un traitement au charbon actif en deux étapes est plus efficace qu'un traitement unique avec double dose, surtout pour l'élimination des peptidoglycanes.

5.2 **Deux traitements en série avec du charbon actif de qualité différente.**

Premier traitement : Noir chimique : ENO-PC de NORIT à 0,25 % de type « mésopore » ;
Deuxième traitement de finition : NORIT SX + à 0,25 % de type « micropore ».

Le tableau XII suivant présente les résultats obtenus. En témoin, un traitement unique au charbon actif avec mélange des deux qualités de charbon actif a également été réalisé (référencé TN « mix »).

Tableau XII

| Hydrolysat d'amidon | | Endotoxines + β-glucanes (EU/g) | Peptidoglycanes (ng/g) |
|---|---|---|---|
| lot « A-5250 » | départ | 9, 6 | 3.540 |
| | 1er TN<br>2ème TN | 0,6<br>< 0,3 | 299<br>< 10 |
| | TN « mix » | < 0,3 | 37 |
| Lot « B-3063 » | Départ | 2,4 | 46.367 |
| | 1er TN<br>2ème TN | < 0,3<br>< 0,3 | 1096<br>< 10 |
| | TN « mix » | < 0,3 | 91 |
| Lot « C » | Départ | 9, 6 | 16.228 |
| | 1er TN<br>2ème TN | 1,2<br>< 0,3 | 777<br>11 |
| | TN « mix » | < 0,3 | 68 |

Il apparaît ici également qu'un traitement en deux étapes est plus efficace qu'un traitement en une seule étape, mélangeant les deux qualités de charbon actif.

Pour le lot A-5250, il apparaît que le premier traitement mésopore permet un abattement plus efficace que le traitement micropore, assurant une finition par traitement micropore qui garantit un taux de contaminants inférieur au seuil de quantification des méthodes classiques de dosage (conformément à l'enseignement de l'exemple 2).

Pour les lots 3063 et C, comme il avait été trouvé dans l'exemple 2 : un double traitement avec la qualité « micropore » NORIT SX + est le plus efficace.

**Revendications**

1.    Procédé de décontamination des hydrolysats d'amidon à partir desquels seront préparés des polymères de glucose destinés à la fabrication de solutions pour dialyse péritonéale, **caractérisé en ce qu'**il comprend les étapes suivantes :

1) préparer un hydrolysat d'amidon par hydrolyse enzymatique ou chimique d'amidon de manière à atteindre un équivalent Dextrose (DE) inférieur à 20, ou par hydrolyse par voie acide d'un lait d'amidon cireux jusqu'à un D.E.

compris entre 8 et 15,

2) filtrer ledit hydrolysat d'amidon de manière à éliminer tout contaminant présentant la taille d'un microorganisme de type levures, moisissures ou bactéries, soit à l'aide d'une filtration membranaire de diamètre de pore de 0,22 $\mu$m, optionnellement précédé d'une préfiltration membranaire d'un diamètre de pore de 0,45 $\mu$m, soit à l'aide d'une ultrafiltration sur membrane présentant un seuil de coupure de 300.000 Da,

3) traiter ledit hydrolysat d'amidon ainsi débarrassé des microorganismes contaminants par une enzyme de dégradation des constituants polysaccharidiques des parois cellulaires, la laminarinase, la laminarinase étant introduite dans les hydrolysats d'amidon à la concentration de 0.001 ‰ à 1 % sur sec en poids d'hydrolysats d'amidon, et étant mise en œuvre à 10% de matière sèche à une température de 50°C, à un pH de 4,6, pendant 5 à 24 heures,

4) ultrafiltrer l'hydrolysat d'amidon ainsi traité enzymatiquement avec une membrane ayant un seuil de coupure de 20.000 Da à 50.000 Da,

5) traiter le perméat d'ultrafiltration contenant l'hydrolysat d'amidon ainsi obtenu sur charbon actif à haute capacité d'adsorption,

6) collecter l'hydrolysat d'amidon ainsi décontaminé.

**2.** Procédé selon la revendication 1, **caractérisé en ce que** l'étape 4) d'ultrafiltration consiste en une ultrafiltration membranaire d'un seuil de coupure de 30.000 Da.

**3.** Procédé selon larevendication 1 ou 2, **caractérisé en ce que** l'étape 5) de traitement au charbon actif à haute capacité d'adsorption consiste en une qualité de charbon actif de type micropore.

**Patentansprüche**

**1.** Verfahren zur Dekontamination von Stärkehydrolysaten, aus denen Glukosepolymere zur Herstellung von Lösungen für die Peritonealdialyse hergestellt werden sollen, **dadurch gekennzeichnet, dass** es die folgenden Schritte umfasst:

1) Herstellen eines Stärkehydrolysats durch enzymatische oder chemische Hydrolyse von Stärke, um ein Dextroseäquivalent (DE) von weniger als 20 zu erreichen, oder durch Säurehydrolyse einer Wachsstärkemilch bis zu einem DE zwischen 8 und 15,

2) Filtrieren des Stärkehydrolysats, um alle Verunreinigungen zu entfernen, die die Größe eines Mikroorganismus vom Typ Hefe, Schimmelpilz oder Bakterium aufweisen, entweder mittels Membranfiltration mit einem Porendurchmesser von 0,22 $\mu$m, dem optional eine Membranvorfiltration vorausgeht, mit einem Porendurchmesser von 0,45 $\mu$m, oder mittels einer Membranultrafiltration mit einer Trenngrenze von 300.000 Da,

3) Behandeln des so von kontaminierenden Mikroorganismen befreiten Stärkehydrolysats mit einem Enzym zum Abbau der Polysaccharidbestandteile der Zellwände, der Laminarinase, wobei die Laminarinase in den Stärkehydrolysaten in einer Konzentration von 0,001 % bis 1 % bezogen auf das Trockengewicht der Stärkehydrolysate eingebracht und bei 10 % Trockensubstanz bei einer Temperatur von 50 °C und einem pH-Wert von 4,6 während 5 bis 24 Stunden eingesetzt wird,

4) Ultrafiltrieren des so enzymatisch behandelten Stärkehydrolysats mit einer Membran mit einer Trenngrenze von 20.000 Da bis 50.000 Da,

5) Behandeln des das so erhaltene Stärkehydrolysat enthaltenden Ultrafiltrationspermeats auf Aktivkohle mit hoher Adsorptionskapazität,

6) Auffangen des so dekontaminierte Stärkehydrolysats.

**2.** Verfahren nach Anspruch 1, **dadurch gekennzeichnet, dass** Schritt 4) der Ultrafiltration in einer Membranultrafiltration mit einer Trenngrenze von 30.000 Da besteht.

**3.** Verfahren nach Anspruch 1 oder 2, **dadurch gekennzeichnet, dass** Schritt 5) der Behandlung mit Aktivkohle mit hoher Adsorptionskapazität eine Aktivkohlequalität vom Mikroporen-Typ aufweist.

**Claims**

**1.** A method for decontaminating starch hydrolysates from which glucose polymers for producing peritoneal dialysis solutions will be prepared, **characterized in that** it comprises the following steps:

1) preparing a starch hydrolysate prepared by enzymatic or chemical hydrolysis of starch so as to achieve a dextrose equivalent (DE) of less than 20, or prepared by acid hydrolysis of a waxy starch milk to give a DE between 8 and 15,

2) filtering said starch hydrolysate so as to remove any contaminant having the size of a microorganism of yeast, mold or bacteria type, either carried out by means of a membrane filtration where the pore diameter is 0,22 $\mu$m, optionally preceded by a membrane filtration where the pore diameter is 0,45 $\mu$m, or carried out by means of a membrane of ultrafiltration where the cut-off threshold is 300.000 Da

3) treating said starch hydrolysate which the contaminating microorganisms have thus been removed by an enzyme for degrading the cell wall polysaccharide constituents, the laminarinase, the laminarinase being introduced into the starch hydrolysates at the concentration of 0,001 ‰ to 1% on a dry weight basis of starch hydrolysates, and being used at 10% of dry mass at a temperature of 50°C, at a pH of 4,6, for 5 to 24 hours,

4) ultrafiltering the starch hydrolysate thus enzymatically treated with a membrane where the cut-off threshold is from 20.000 Da to 50.000 Da,

5) treating the resulting ultrafiltration containing the starch hydrolysate on activated carbon with a high adsorption capacity,

6) collecting the starch hydrolysate thus decontaminated.

2. The method as claimed in claim 1, **characterized in that** the ultrafiltration of step 4) consists of a membrane of ultrafiltration where the cut-off threshold is 30.000 Da.

3. The method as claimed in claim 1 or 2, **characterized in that** step 5) of treating with activated carbon with a high adsorption capacity consists of a quality of activated carbon of "micropore" type.

## RÉFÉRENCES CITÉES DANS LA DESCRIPTION

**Documents brevets cités dans la description**

- EP 207676 A
- EP 667356 A
- WO 2007099212 A
- EP 1720999 A
- WO 2009117302 A
- WO 2009117303 A
- WO 2009117304 A
- WO 2009117558 A, BAXTER